Europäisches Patentamt

European Patent Office

Office européen des brevets

(19)

(11) Veröffentlichungsnummer: **0 077 537**
**B1**

(12) **EUROPÄISCHE PATENTSCHRIFT**

(45) Veröffentlichungstag der Patentschrift:
02.10.85

(51) Int. Cl.⁴: **C 07 C 143/63,** C 07 C 139/00

(21) Anmeldenummer: 82109543.7

(22) Anmeldetag: 15.10.82

(54) Verfahren zur Herstellung von 6-Chlor-3-toluidin-4-sulfonsäure.

(30) Priorität: 17.10.81 DE 3141286

(43) Veröffentlichungstag der Anmeldung:
27.04.83 Patentblatt 83/17

(45) Bekanntmachung des Hinweises auf die Patenterteilung:
02.10.85 Patentblatt 85/40

(84) Benannte Vertragsstaaten:
BE CH DE FR GB IT LI

(56) Entgegenhaltungen:
Patent Abstracts of Japan
Chemical Abstracts Band 87, Nr. 19, 7. November 1977,
Columbus, Ohio, USA
Patent Abstracts of Japan
HOUBEN WEYL "Methoden der Organischen Chemie",
4. Auflage 1971, GEORG THIEME VERLAG, Stuttgart,
Band X/1, Seiten 552-557
HOUBEN WEYL "Methoden der Organischen Chemie",
4. Auflage 1955, GEORG THIEME VERLAG, Stuttgart,
Band IX, Seiten 463-470

(73) Patentinhaber: HOECHST AKTIENGESELLSCHAFT,
Postfach 80 03 20, D-6230 Frankfurt am Main 80 (DE)

(72) Erfinder: Papenfuhs, Theodor, Dr.,
Heinrich-Bleicher-Strasse 40, D-6000 Frankfurt am
Main 50 (DE)
Erfinder: Berthold, Rüdiger, Dr., Geierfeld 55,
D-6232 Bad Soden am Taunus (DE)

0 077 537

## Beschreibung

6-Chlor-3-toluidin-4-sulfonsäure (5-Amino-2-chlor-toluol-4-sulfonsäure) und ihre Salze sind aufgrund ihrer unterschiedlichen reaktiven Gruppen vielseitig verwendbare Zwischenprodukte, die insbesondere zur Herstellung von Farbmitteln Verwendung finden.

Es sind bereits eine Reihe von Verfahren zur Herstellung von 5-Amino-2-chlor-toluol-4-sulfonsäure beschrieben, so beispielsweise die Herstellung aus Toluol (BIOS Final Report Nr. 986/1; No. 76, S. 130—134), aus 2-Chlor-toluol-4-sulfonsäure (DE-PS 145 908, Friedl. 7 (1902/04)467), aus m-Xylol (JP-OS 52/62 239 und 52/65 245, C. A. 87 (1977), Nr. 117 678 und Nr. 151 849) sowie aus o-Chlortoluol (JP-OS 54/151 945, C. A. 92 (1980), Nr. 198 110). Die vom m-Xylol ausgehenden Wege erfordern eine Vielzahl von Reaktionsstufen mit teilweise unbefriedigenden Ausbeuten und unerwünschten Nebenprodukten, die diese Verfahren außerordentlich aufwendig gestalten. Dasselbe gilt für das vom Toluol ausgehende Verfahren, das in zwei Reaktionsschritten zur 2-Chlor-toluol-4-sulfonsäure führt, die das Ausgangsmaterial für das in der DE-PS 145 908 beschriebene Verfahren darstellt, welches erst in zwei weiteren Schritten zum gewünschten Produkt führt. Hierbei werden in allen Stufen unerwünschte, technisch nicht nutzbare Isomere gebildet, was zu einer Ausbeute am erwünschten Endprodukt von maximal 50% führt. Die Abtrennung und insbesondere Beseitigung der unerwünschten Nebenprodukte ist außerordentlich aufwendig, da die anfallenden Mutterlaugen verbrannt werden müssen.

Am einfachsten erscheint das in der JP-OS 54/151 945 beschriebene Verfahren, das von o-Chlortoluol ausgeht. Dieses Ausgangsmaterial wird zum 5-Brom-2-chlor-toluol bromiert, zum 5-Amino-2-chlortoluol aminiert, das in einer »Backreaktion« (vgl. ABC Chemie, Bd. 1, Frankfurt/M.—Zürich, 1965, S. 153; Ullmanns Encyklopädie der technischen Chemie, 4. Aufl., Bd. 8, Weinheim, 1974, S. 246) zum gewünschten Produkt umgesetzt wird. Allerdings verläuft die Bromierung nicht einheitlich, so daß eine aufwendige Abtrennung und Beseitigung der unerwünschten Isomeren erforderlich wird. Hinzu kommt der Aufwand, der mit der Rückgewinnung des Broms aus dem in der ersten Stufe gebildeten Bromwasserstoff und dem in der zweiten Stufe gebildeten Ammoniumbromid verbunden ist.

Es wurde nun gefunden, daß man die 6-Chlor-3-toluidin-4-sulfonsäure bzw. deren Natriumsalz in einfacher Weise und ohne Anfall unerwünschter Nebenprodukte herstellen kann, indem man das o-Chlortoluol mit Mischsäure nitriert, das 2-Chlor-5-nitro-toluol von seinen Isomeren abtrennt und reduziert und das erhaltene 5-Amino-2-chlor-toluol mit der äquimolaren Menge Schwefelsäure oder Natriumhydrogensulfat — mit oder ohne anschließender Isolierung des intermediär gebildeten Aminhydrogensulfats bzw. Amin-Natrium-sulfats — durch Erhitzen auf Temperaturen über 180°C zur 6-Chlor-3-toluidin-4-sulfonsäure bzw. zu deren Natriumsalz verbackt.

Die Mischsäure-Nitrierung von o-Chlortoluol ist an sich bekannt (Rec. Trav. Chim. Pays-Bas 32 [1913], 244 ff., insbes. 247ff.). Von den hierbei entstehenden Isomeren ist das erwünschte 2-Chlor-5-nitro-toluol durch Destillation in 43%iger Ausbeute zu isolieren (a.a.o. 286). Als Vorlauf fällt hierbei reines 2-Chlor-6-nitro-toluol an; die restlichen Isomeren sind in bekannter Weise zu trennen. Da alle Isomeren aufgrund ihrer unterschiedlichen reaktiven Gruppen vielseitig einsetzbare Zwischenprodukte darstellen, fallen bei dieser Reaktion keine unerwünschten Nebenprodukte an.

Die Reduktion des 2-Chlor-5-nitro-toluols zum 5-Amino-2-chlor-toluol erfolgt in der für Halogen-Nitroaromaten üblichen Weise, beispielsweise mit Eisen und Säure, vorzugsweise jedoch durch katalytische Hydrierung, beispielsweise nach dem Verfahren der DE-PS 1 959 578 mit sulfidierten Platinkatalysatoren oder besonders vorteilhaft nach dem Verfahren der DE-PS 2 105 780 mit sulfitierten Platinkatalysatoren. Hierbei kann die Reduktion in Wasser oder in einem organischen Lösungsmittel durchgeführt werden.

Die Sulfierung des 5-Amino-2-chlor-toluols erfolgt durch Erhitzen seines sauren Sulfats auf erhöhte Temperatur, wobei unter Wasserabspaltung die freie 6-Chlor-3-toluidin-4-sulfonsäure gebildet wird. Diese Reaktion kann sowohl durch trockenes Erhitzen (»Verbacken«, vgl. Ullmann, l.c., Kirk-Othmer, Encyclopedia of Chemical Technology, 2. Aufl., Bd. 2, New York—London—Sydney 1963, S. 424), als auch in Gegenwart indifferenter Verdünnungsmittel, beispielsweise o-Dichlorbenzol, durchgeführt werden, wobei das gebildete Wasser abdestilliert oder verdampft, vorteilhaft in Form eines Azeotrops mit dem Verdünnungsmittel und/oder durch Arbeiten unter vermindertem Druck.

Das saure Sulfat kann in einem getrennten Schritt, beispielsweise durch Vermischen des gegebenenfalls geschmolzenen 5-Amino-2-chlor-toluols mit stöchiometrischen Mengen wäßriger, beispielsweise 60- bis 90%iger, Schwefelsäure in einem Rühr- oder Knetapparat bei Temperaturen oberhalb 120°C hergestellt werden. Die hierbei erhaltene Schmelze kann direkt oder nach Abkühlen und Zerkleinern »verbacken« werden. Die Herstellung des sauren Sulfats kann aber auch in Gegenwart von Verdünnungsmitteln, beispielsweise o-Dichlorbenzol, erfolgen, und — vorzugsweise ohne Zwischenisolierung — anschließend durch Abdestillieren des Reaktionswassers in einer Eintopfreaktion zur 6-Chlor-3-toluidin-4-sulfonsäure umgesetzt werden.

Zum Nariumsalz der 6-Chlor-3-toluidin-4-sulfonsäure gelangt man durch »Verbacken« des 5-Amino-2-chlor-toluols mit äquivalenten Mengen Natriumhydrogensulfat, während durch »Verbacken« des aus Wasser kristallisierenden neutralen Sulfats des 5-Amino-2-chlor-toluols mit einem halben Mol Natriumhydrogensulfat ein Gemisch aus der freien Säure und ihrem Natriumsalz gebildet wird.

Die »Backreaktion« erfolgt durch Erhitzen auf Temperaturen über 180°C. Eine bevorzugte Ausführungsform der »Backreaktion« besteht im Erhitzen in einem Kneter, z. B. in einem sogenannten »Allphasenreaktor« nach dem Verfahren der DE-OS 2 439 297.

Das erfindungsgemäße Verfahren erlaubt es somit, die 6-Chlor-3-toluidin-4-sulfonsäure wesentlich einfacher als nach den bisher bekannten Verfahren herzustellen, wobei noch dazu keine unerwünschten Nebenprodukte anfallen. Die Produkte werden in einer Reinheit erhalten, daß sie ohne weitere Reinigung zur Herstellung von Farbmitteln dienen können.

In den folgenden Beispielen beziehen sich Prozentangaben auf das Gewicht, sofern nichts anderes angegeben ist.

### A) Herstellung des 5-Amino-2-chlor-toluols aus 2-Chlor-5-nitro-toluol

### Beispiel 1

In einem 2 l fassenden Hydrierer wurde eine Suspension von 172 g 2-Chlor-5-nitro-toluol (1 Mol) und 500 ml Wasser in Gegenwart von 1 g eines sulfitierten Platinkatalysators (nach Beispiel 1 der DE-PS 2 105 780) solange mit Wasserstoff behandelt, bis keine Druckabnahme mehr erfolgte. Die Reaktion begann bei 50°C und wurde bei einem Wasserstoffdruck von 40 bar bei Temperaturen zwischen 80 und 100°C innerhalb einer Stunde zu Ende geführt. Die Suspension wurde bei 95 bis 100°C über eine dampfbeheizte Drucknutsche filtriert und das Filtrat bis zum Erkalten gerührt. Der abgetrennte Katalysator kann unmittelbar für eine folgende Reaktion wieder eingesetzt werden. Aus dem erkalteten Filtrat isolierte man durch Absaugen 135 g 98,5%iges 5-Amino-2-chlor-toluol vom Fp. 82—83°C, entsprechend 94% d. Th.

### Beispiel 2

172 g 2-Chlor-5-nitro-toluol wurden in 350 ml Methanol gelöst und in einem 1-l-Hubrührautoklav in Gegenwart von 2 g des in Beispiel 1 eingesetzten sulfitierten Platinkatalysators bei etwa 80°C mit Wasserstoff hydriert. Nach Aufnahme der berechneten Wasserstoffmenge rührte man noch 10 Minuten nach und ließ die Reaktionsmischung erkalten. Nach Absaugen des Katalysators wurde aus dem Filtrat das Methanol abdestilliert. Man erhielt 137 g 5-Amino-2-chlor-toluol von 98%iger Reinheit, entsprechend 95% der Theorie.

### Beispiel 3

250 g Eisenspäne wurden in 300 ml Wasser vorgelegt und mit 20 ml Essigsäure angeätzt. Nach Zugabe von 400 ml Wasser wurde auf 80°C erwärmt und 2 g 2,4,6-Tritertiär-butylphenol (als Netzmittel) zugegeben. Nach Erhitzen auf 100°C trug man nach und nach 172 g 2-Chlor-5-nitro-toluol so ein, daß die Reaktionsmischung ständig siedete. Der pH-Wert der Mischung wurde im Bereich von 6,0—6,5 gehalten. Ein eventuelles Schäumen wird durch Zugabe von wenig Tributylphosphat bekämpft. Nachdem des gesamte 2-Chlor-5-nitro-toluol zugegeben war, erhitzte man noch solange unter Rückfluß, bis keine Nitroverbindung mehr nachweisbar war (etwa 30 Minuten). Durch Zugabe von Natronlauge wurde der pH auf 8—9 eingestellt und die siedende heiße Reduktionsmischung über eine dampfbeheizte Drucknutsche filtriert. Der Eisenschlamm wurde mit 200 ml siedendem Wasser gewaschen. Nach dem Erkalten des Filtrats wurde das auskristallisierte 5-Amino-2-chlor-toluol abgesaugt. Man erhielt 128,5 g 98%iges Produkt, entsprechend 89% d. Th.

### B) Herstellung der 5-Amino-2-chlor-toluol-4-sulfonsäure

### Beispiel 4

In einem 3-l-Planflanschbecher mit gut wandgängigem Ankerrührer wurden 71 g 5-Amino-2-chlor-toluol (0,5 Mol) und 175 ml o-Dichlorbenzol vorgelegt. Unter Rühren ließ man innerhalb von 10 Minuten 54 g 100%ige Schwefelsäure zulaufen, wobei die Innentemperatur auf etwa 100°C anstieg. Die entstandene dicke weiße Suspension wurde weiter erhitzt und durch Sieden unter Rückfluß mittels eines Wasserabscheiders das Reaktionswasser ausgekreist. Nach etwa 5 Stunden waren 9 ml Wasser übergegangen, was der theoretischen Menge entspricht. Nach Abkühlen auf 80°C gab man 30 ml Wasser zu und stellte durch Zugabe von 80 g 33%iger Natronlauge alkalisch. Nach Abdestillieren des o-Dichlorbenzols mit Wasserdampf wurde die heiße Lösung mit 10 g Aktivkohle geklärt. Das Filtrat wurde mit Salzsäure auf pH 7 eingestellt und Kochsalz zugesetzt. Nach Absaugen bei 10°C erhielt man 118 g Natriumsalz der 5-Amino-2-chlor-toluol-4-sulfonsäure in 98%iger Reinheit, entsprechend 95% d. Th.

**0 077 537**

### Beispiel 5

In einen auf etwa 180° C geheizten Labor-Kneter (Sigma-Kneter der Firma Werner & Pfleiderer) legte man 60 g 80%ige Schwefelsäure vor und trug anschließend 71 g geschmolzenes 5-Amino-2-chlor-toluol (0,5 Mol) ein. Es entstand eine dunkelbraune Schmelze des sauren Sulfats, die im Verlauf des Knetvorgangs fest und krümelig wurde. Man erhielt 125 g 5-Amino-2-chlor-toluol-Hydrogensulfat. Dieses Salz wurde 6 Stunden bei 220° C auf einem Blech im Trockenschrank verbacken. Man erhielt 113 g einer schwarzen Rohbackschmelze, die aus verdünnter Natronlauge umgelöst wurde. Durch Fällen mit Salzsäure erhielt man 115 g Natriumsalz der 5-Amino-2-chlor-toluol-4-sulfonsäure, entsprechend 91% d. Th.

### Beispiel 6

142 g 5-Amino-2-chlor-toluol (1 Mol) wurden mit 125 g Natriumhydrogensulfat gut vermahlen und wie in Beispiel 5 angegeben 6 Stunden verbacken. Man erhielt 234 g schwarze Rohbackschmelze, die aus 1,5 l Wasser unter Zusatz von Aktivkohle umgelöst wurde. Nach Aussalzen mit Kochsalz erhielt man 214 g Natriumsalz der 5-Amino-2-chlor-toluol-4-sulfonsäure, entsprechend 88% d. Th.

### Beispiel 7

142 g 5-Amino-2-chlor-toluol wurden in 750 g 7,5%iger Schwefelsäure heiß gelöst, die Lösung mit Aktivkohle geklärt und nach dem Abkühlen das ausgefallene 5-Amino-2-chlor-toluol-Sulfat abgesaugt und getrocknet. Von diesem neutralen Salz wurden 95,5 g (0,5 Mol) mit 62,5 g Natriumhydrogensulfat vermahlen und, wie in Beispiel 6 beschrieben, verbacken und umgelöst. Man erhielt 108,5 g Natriumsalz der 5-Amino-2-chlor-toluol-4-sulfonsäure, entsprechend 89% der Theorie.

### Beispiel 8

56,6 kg 5-Amino-2-chlor-toluol und 40,8 kg Schwefelsäure, jeweils je Stunde, wurden kontinuierlich in eine Mischapparatur eingepumpt und das erhaltene 5-Amino-2-chlor-toluol-bisulfat in einen kontinuierlich arbeitenden Allphasenreaktor (Typ 125 R), der ein Reaktionsvolumen von etwa 70 l hatte und in dem ein reduzierter Druck von etwa 25 mbar aufrechterhalten wurde, eingebracht. Die Heizflächen des Reaktors wurden mit erhitztem Öl geheizt, und zwar so, daß in der ersten Reaktionszone eine Innentemperatur von etwa 210° C und in der zweiten eine solche von etwa 240° C herrschte. Das 5-Amino-2-chlor-toluol-bisulfat setzte sich unter Wasserabspaltung zu 5-Amino-2-chlor-toluol-4-sulfonsäure um. Der entstandene Wasserdampf (7,2 kg je Stunde) verließ den Reaktor über ein Brüdenrohr und wurde anschließend kondensiert. Am Ausgang verließen 88,6 kg je Stunde 5-Amino-2-chlor-toluol-4-sulfonsäure mit einer Reinheit von 98% den Reaktor.

### Beispiel 9

In einen wie in Beispiel 8 beschriebenen kontinuierlich arbeitenden Allphasenreaktor pumpte man je Stunde 42,5 kg 5-Amino-2-chlor-toluol und 30,6 kg Schwefelsäure. In der ersten Reaktionszone bildete sich bei einer Innentemperatur von 180—220° C 5-Amino-2-chlor-toluol-bisulfat. Dieses setzte sich in der zweiten Reaktionszone (Innentemperatur etwa 240° C) unter Wasserabspaltung zu 5-Amino-2-chlor-toluol-4-sulfonsäure um. Diese Umsetzung wurde in der dritten Reaktionszone (Innentemperatur etwa 260° C) vervollständigt. Der entstandene Wasserdampf (5,4 kg je Stunde) verließ den Reaktor über ein Brüdenrohr und wurde anschließend kondensiert. Am Ausgang verließen 66,5 kg 5-Amino-2-chlor-toluol-4-sulfonsäure mit einer Reinheit von 95—96% den Reaktor.

**Patentansprüche**

1. Verfahren zur Herstellung von 6-Chlor-3-toluidin-4-sulfonsäure aus o-Chlortoluol über die Stufe des 5-Amino-2-chlor-toluols und Verbacken desselben, dadurch gekennzeichnet, daß man das o-Chlortoluol mit Mischsäure nitriert, das 2-Chlor-5-nitro-toluol von seinen Isomeren abtrennt und reduziert, und das erhaltene 5-Amino-2-chlor-toluol mit der äquimolaren Menge Schwefelsäure oder Natriumhydrogensulfat — mit oder ohne anschließender Isolierung des intermediär gebildeten Aminhydrogensulfats bzw. Amin-Natrium-sulfats — durch Erhitzen auf Temperaturen über 180° C zur 6-Chlor-3-toluidin-4-sulfonsäure bzw. zu deren Natriumsalz verbackt.

2. Verfahren nach Anspruch 1, dadurch gekennzeichnet, daß die Reduktion durch katalytische

4

Hydrierung erfolgt.

3. Verfahren nach Anspruch 1 und 2, dadurch gekennzeichnet, daß das Verbacken in einem Kneter erfolgt.

4. Verfahren nach Anspruch 1 bis 3, dadurch gekennzeichnet, daß die Reduktionslösung im schwefelsauren Medium aufgearbeitet wird und das dabei erhaltene Produkt zusammen mit Natriumhydrogensulfat verbacken wird.

5. Verfahren nach Anspruch 1, dadurch gekennzeichnet, daß man das 5-Amino-2-chlor-toluol in Form des neutralen Sulfats mit der halben molaren Menge Natriumhydrogensulfat verbackt.

## Claims

1. A process for the preparation of 6-chloro-3-toluidine-4-sulfonic acid from o-chlortoluene via the stage of 5-amino-2-chlorotoluene and »baking« the latter, which comprises nitrating the o-chlorotoluene with mixed acid, separating the 2-chloro-5-nitro-toluene from its isomers and reducing it, and baking the obtained 5-amino-2-chloro-toluene with the equimolar amount of sulfuric acid or sodiumhydrogensulfate — with or without subsequent isolation of the intermediarily formed aminohydrogensulfate or amine — sodiumsulfate respectively, by heating to temperatures above 180°C to 6-chloro-3-toluidine-4-sulfonic acid or to its sodium salt respectively.

2. The process as claimed in Claim 1, which comprises carrying out the reduction by catalytic hydrogenation.

3. The process as claimed in Claims 1 and 2, which comprises carrying out the baking in a kneader.

4. The process as claimed in Claim 1 to 3, which comprises working up the reduction solution in a medium of sulfuric acid, and baking the product so obtained together with sodium hydrogensulfate.

5. The process as claimed in Claim 1, which comprises the baking of 5-amino-2-chloro-toluene in form of the neutral sulfate with the half molar amount of sodiumhydrogensulfate.

## Revendications

1. Procédé pour préparer l'acide chloro-6 toluidine-3 sulfonique-4 à partir de l'o-chloro-toluène en passant par le chloro-2 amino-5 toluène et en cuisant celui-ci, procédé caractérisé en ce qu'on nitre l'o-chloro-toluène au moyen d'un mélange sulfo-nitrique, on sépare le chloro-2 nitro-5 toluène de ses isomères, on le réduit et on cuit le chloro-2 amino-5 toluène ainsi obtenu avec la quantité équimolaire d'acide sulfurique ou d'hydrogéno-sulfate de sodium — avec ou sans isolement ultérieur de l'hydrogéno-sulfate d'amine ou du sulfate de sodium et d'amine intermédiairement formé — par chauffage à des températures supérieures à 180°C, de manière à obtenir l'acide chloro-6 toluidine-3 sulfonique-4 ou son sel de sodium.

2. Procédé selon la revendication 1, caractérisé en ce qu'on effectue la réduction par hydrogénation catalytique.

3. Procédé selon l'une des revendications 1 et 2, caractérisé en ce qu'on effectue la cuisson dans un malaxeur.

4. Procédé selon l'une quelconque des revendications 1 à 3, caractérisé en ce qu'on traite la solution de réduction dans un milieu acidifié par de l'acide sulfurique et on cuit le produit ainsi obtenu avec de l'hydrogéno-sulfate de sodium.

5. Procédé selon la revendication 1, caractérisé en ce qu'on cuit le chloro-2 amino-5 toluène sous la forme du sulfate neutre avec la moitié de la quantité molaire d'hydrogéno-sulfate de sodium.